# EUROPEAN PATENT SPECIFICATION

(11) **EP 0 650 516 B1**
(45) Date of publication and mention of the grant of the patent: **30.10.2002**
(21) Application number: 93911189.4
(22) Date of filing: 12.05.1993
(51) Int. Cl.: C10G 45/40, C07C 7/167

(54) **Arsine as moderator in the conversion of acetylene**
Arsin als Moderator für die Acetylenumwandlung
Utilisation de l'arsine en tant que modérateur de conversion de l'acétylène

(30) Priority: 12.05.1992 US 881902
(43) Date of publication of application: 03.05.1995
(73) Proprietor: ExxonMobil Chemical Patents Inc., Baytown, TX 77520-5200 (US)
(72) Inventor: SLIM, David, Robert, Sarnia, Ontario N7S 3Z2 (CA); MOHUNDRO, Edgar Lucian, Baytown, TX 77521 (US); MAYO, Stephen Mark, Stillwater, OK 74075 (US)
(74) Representative: Dew, Melvyn John
(86) International application number: PCT/US93/04388
(87) International publication number: WO 93/023499

(56) References cited:
- EP-A- 0 334 742
- US-A- 2 837 587
- US-A- 3 697 448
- US-A- 3 900 526
- US-A- 4 227 025
- US-A- 4 593 148
- US-A- 4 605 812

## Description

The present invention relates to the removal of acetylene from hydrocarbon streams; particularly but not exclusively to the production of ethylene which is substantially free of acetylene; and especially to minimizing the contamination of ethylene by acetylene produced by stream cracking. In an especially preferred aspect, the present invention is directed to selective acetylene hydrogenation over a palladium catalyst using arsine or phosphine as an acetylene converter moderator.

In the production of hydrocarbons such as ethylene, the presence of acetylene is undesirable, but one of the by-products from streamcracking is acetylene. Therefore, there has been a general concern for minimizing the presence of acetylene in ethylene. Conventional procedures which have been proposed to eliminate the deleterious effects of the presence of acetylene in ethylene include the selective hydrogenation of acetylene over a palladium catalyst.

Inasmuch as acetylene hydrogenation is a highly exothermic reaction, it has been necessary to add a moderator during the reaction to enhance selectivity. A conventional acetylene converter moderator which is typically employed for this purpose is carbon monoxide, as discussed in United Catalysts Inc.'s recent "Quality Products & Services" brochure relating to palladium catalyst systems on page 5. Normally, carbon monoxide is added at concentrations of from 1 ppm -5 ppm, and acts as a temporary poison.

It has been observed that the carbon monoxide does not remain on the catalyst surface but ends up in the product ethylene. This is unacceptable inasmuch as carbon. monoxide is a recognized poison and carbon monoxide contamination of product ethylene can be a problem for downstream polymerization processes, e.g., in the production of high density polyethylene (HDPE) and linear low density polyethylene (LLDPE).

The present invention is based on the identification of acetylene converter moderators which are substances which remain on the catalyst surface and do not migrate into the product ethylene stream. The acetylene converter moderators required for purposes of the present invention are arsine and phosphine, more preferably arsine. The following prior art is therefore discussed with reference to the use of arsine as moderator.

It is generally known that high levels of arsine tend to poison acetylene converter catalysts.

US-A-4,227,025 (Phillips Petroleum Co.) is directed to a process for the removal of acetylene from cracked gases obtained from conversion processes in which crude oil containing minor amounts of arsenic are thermally or catalytically cracked, hydrocracked, or otherwise subjected to modification resulting in the production of gases containing arsenic. More specifically, arsenic-poisoned palladium catalysts may be regenerated in place by providing a purging step with an arsenic-free gas under acetylene removal conditions into contact with the hydrogenation catalyst, for example, a noble metal catalyst, such as palladium, until the activity and selectivity for removal of acetylene has been restored to the catalyst.

It is also known to hydrogenate acetylenes using homogeneous catalysts that involve phosphine or arsine ligands.

US-A-4,268,454 (Allied Chemical Corp.) discloses that complexes of anionic group VIII metal (first or second transition series), and hybrid complexes having 1-3 ligands per metal atom, are useful as catalysts in the homogeneous hydrogenation of aldehydes, ketones, olefins and alkynes.

Other publications which generally teach the hydrogenation of unsaturated hydrocarbons, especially in the presence of transition metal catalysts comprising or associated with arsine or phosphine containing ligands, include: US-A-4,645,849 (General Electric Co.); US-A-3,574,716; GB-A-1,121,643; GB-A-1,154,937; US-A-3,463,830; GB-A-1,285,871 (all Imperial Chemical Industries, Ltd.); GB-A-1,182,353; US-A-3,453,302 (both Montecatini Edison S.p.A.); US-A-3,697,615 (Phillips Petroleum Co.); FR-A-2,588,197 (Poudres & Explosifs); EP-A-66,287 (Wacker Chemie GmbH); GB-A-2,171,719; US-A-3,917,737; and GB-A-1,378,747. Thus, the use of phosphines and arsines in complexes used for hydrogenation appear to be generally known inasmuch as such ligands confer essential stability on the complex.

US-A-4,377,503 (Dessau) is directed to a shape-selective zeolitic catalyst useful for various purposes, including hydrogenation of acetylenes. Dessau discloses that shape-selective characteristics can be imparted to the metal containing catalysts by reducing a metal containing zeolite in the presence of one or more unsaturated compounds; and that selectivity can be increased, for example, by means of high temperature hydrogen treatment of the zeolite. Shape selectivity of the catalyst can be increased by selectively poisoning the catalyst with a "bulky" poison, such as tri-p-tolylphosphine, which is described as being larger than the pore size of the zeolite, and thus deactivates the metallic catalyst component on the "outside" of the catalyst composite to a greater extent than such poisons would poison the metal function in the interstices of the composite.

In general, however, arsine is considered to be a poison for catalysts used in selective hydrogenation of acetylenes.

As indicated above, US-A-4,227,025 is directed to the removal of acetylene from ethylene using catalysts which are periodically reactivated after being poisoned by the arsenic.

US-A-4,605,812 (Phillips Petroleum Co.) is also directed to removing arsenic impurities, such as hydrocarbyl arsines, from hydrocarbon streams in order to remove the arsine impurities from the hydrocarbon stream effluent with a noble metal hydrogenation catalyst to hydrogenate the olefin present therein.

US-A-4,593,148 (Phillips Petroleum Co.) is directed to the removal of arsine from gases, for example, by contacting with sorbent comprising copper oxide and zinc oxide, to remove arsine impurities for the purpose of preventing catalyst poisoning by AsH₃ and/or hydrocarbyl arsines in a hydrocarbon feedstream, for example, C₂₋₆ olefin streams, prior to hydrogenation of acetylenic impurities on noble metal catalysts.

Methods and apparatus referred to herein include:

An MDA analyzer model 7100 supplied commercially by MDA Scientific, Lincolnshire, Illinois, USA, which is a continuous toxic gas monitor which, in the Serials 7100, is disclosed as being useful for ammonia, chlorine, diisocyanates, hydrazines, hydrides, hydrogen chloride, hydrogen cyanide, hydrogen fluoride, hydrogen sulfide, nitric acid, nitrogen dioxide, pphenylene diamine, phosgene, and sulfur dioxide. As advertised, the Series 7100 continuous toxic gas monitors are capable of detecting concentrations as low as 1 ppb.

The British standard method for the determination of arsenic which is disclosed in British Standards Publication BS4404 and which is also referred to as the silver diethyldithiocarbamate procedure for the determination of arsenic.

### SUMMARY OF THE INVENTION

The present invention is based on the discovery that certain acetylene converter moderators, being arsine and phosphine, can be used to moderate the activity of acetylene hydrogenation catalysts while maintaining acceptable catalyst activity, enhancing the acetylene hydrogenation selectivity to ethylene, and minimizing the risk of product quality contamination.

Thus, according to the present invention there is provided a process for removing acetylene from a hydrocarbon stream, said stream comprising an acetylene converter moderator, by contacting the stream with an acetylene hydrogenation catalyst under conditions effective to permit acetylene hydrogenation, characterized in that the moderator is arsine or which is introduced into the stream and controlled to a concentration of from 1 to 5 wppb prior to contact with the catalyst. The acetylene converter moderator which substantially remains on the catalyst and does not substantially migrate into the stream.

Preferably the moderator comprises arsine. Since it is generally known that arsine tends to poison acetylene converter catalysts, and indeed since systems are normally put into place to remove arsine from the reaction zone in some fashion, the finding that arsine and phosphine can be effectively used as acetylene converter modifiers, is particularly unexpected.
An important feature of the process is that the moderator substantially remains on the catalyst and does not migrate into the hydrocarbon stream. Since arsine is a particularly effective moderator of this type, the following description is directed principally to the use of arsine.

In this regard, and in accordance with the present invention, the moderator, arsine, may be used at a relatively low level within a concentration range which has been found to be effective for maintaining acceptable catalyst activity and enhancing selectivity to the desired extent. Such concentration is from 1 wppb-5 wppb, preferably from 1-3 wppb. These concentrations render arsine safer to use than conventional acetylene converter moderators, such as carbon monoxide.

It is believed that in the stated concentrations arsine and phosphine function as acetylene converter moderators because they remain on the catalyst, rather than being transferred into the product stream, so that they do not tend to poison downstream, e.g., LLDPE production processes.

In one aspect of the invention, the hydrogenation catalyst is exposed to the moderator arsine, by blending, e.g., up to 1000 wppm of the moderator in a carrier gas, preferably using a direct external injection technique or a controlled leakage technique, most preferably the former. Preferably the amount of moderator, e.g., arsine, contained in the carrier gas is up to 400 ppm, more preferably from 10 ppm-400 ppm, and most preferably about 200 ppm.

The carrier gas may be for example ethylene, ethane, nitrogen, helium, argon or a mixture or one or more thereof, and preferably comprises argon or ethylene.

In preferred processes of the invention, the conditions effective to support acetylene hydrogenation include, independently: a temperature of from 26 to 177°C (80 to 350°F) ; a pressure of from 689 kPa to 5-17 MPa (100-750 psi); and a gas hourly space velocity of from 3000-15000 v/hr/v.

In a preferred embodiment the process of the invention involves contacting the hydrogenation catalyst with carrier gas containing from 100 wppm - 20,000 wppm acetylene, preferably at a temperature of from 26 to 177°C, a pressure of from 689 kPa to 5.17 MPa, and at a gas hourly space velocity of from 3000 v/hr./v. to 15,000 v/hr./v. to control the amount of moderator, e.g., arsine, on the hydrogenation catalyst to within the range of from 1 wppb to 3 wppb.

One method of performing the process of the invention involves regenerating the deactivated catalyst to restore at least some of any loss of catalyst activity, for example due to coking, green oil formation or a combination thereof. The regeneration of the catalyst may be repeated successively until the catalyst activity is reduced to an unacceptable level due to coking, green oil formation, or a combination of coking and green oil formation. For purposes of the present invention, an unacceptable level of catalyst activity or selectivity loss due to coking, green oil formation or the combination of coking and green oil formation occurs before a permanent poisoning by arsenic.

In the process of the invention, the hydrogenation catalyst may be a transition metal selected from the group consisting of Group VIII metals, Group VIIIA metals and IB metals, preferably palladium. The catalyst may be based on a support, in which case the preferred support material comprises silica, zeolite, alumina, or a combination of two or more thereof. The particularly preferred support is alumina.

The catalysts which are particularly suitable for purposes of the present invention include: 0.01 - 0.5 wt % palladium on a support, especially a palladium-type hydrogenation catalyst, for example, palladium on alumina, which is selected for the hydrogenation of acetylene into ethylene in the presence of ethylene.

In the process where the catalyst is palladium supported on alumina, and the moderator is arsine, it is believed that regenerating results in transferring arsenic from palladium sites to the alumina.

In the process of the invention, the hydrocarbon stream comprises, for example, ethylene or a mixture of ethylene and ethane. The stream used in the process may be produced by subjecting hydrocarbon(s) to e.g., steam cracking, catalytic cracking, or coking.

In accordance with one embodiment of the process of the invention, the hydrocarbon stream comprises catalytic cracker gas, steam cracker gas, ethylene and acetylene and is produced by a procedure selected from the group consisting of steam cracking, catalytic cracking, and coking, the conditions effective to support acetylene hydrogenation including a temperature of from 26 to 177°C (80°F to 350°F), pressures of from 689 kPa to 5.17 MPa (100 psi to 750 psi), and hourly space velocities of from 3,000 to 15,000 v/hr./v.

In general, the parameters suitable for purposes of the acetylene removal operation may be conventional. Particularly preferred for purposes of the present invention, however, are temperatures of from 10°C to 177°C (50°F to 350°F).

Other parameters for practicing the invention include the following:

| | Suitable | Preferred |
|---|---|---|
| Inlet Temperature, | 10-260 | 26.7-149 |
| °C (°F) | (50-500) | (80-300) |
| | | |
| GHSV, v/v/hr | 500-15000 | 1500-10000 |
| | | |
| H₂/C₂H₂ | 1.0-10.0 | 1.0-3.0 |

| | More Preferred | Most Preferred |
|---|---|---|
| Inlet Temperature, | 37.7-93.3 | 51.7-65.5 |
| °C (°F) | (100-200) | (125-150) |
| | | |
| GHSV, v/v/hr | 2000-8000 | 4000-6000 |
| | | |
| H₂/C₂H₂ | 1.0-2.0 | 1.0-1.5 |

The process preferably employs hydrogen and hydrocarbon, e.g., ethylene, in a ratio (molar) of from 1:1 - 1:10, more preferably 1:1 - 1:3.

The process of the invention preferably yields a hydrocarbon, e.g., ethylene, product which is substantially devoid of acetylene and substantially devoid of at least one member selected from the group consisting of carbon monoxide, arsine, and phosphine, and is most preferably substantially devoid of carbon monoxide.

The present invention is also directed to a method of producing a further processed hydrocarbon, e.g., ethylene, product which involves providing hydrocarbon, e.g.,ethylene, which is substantially devoid of acetylene and acetylene converter moderators; made by the process of the invention, and subjecting such hydrocarbon, e.g., ethylene, to further processing steps to produce a product comprising or based on said hydrocarbon, e.g., ethylene. The further processing may include for example, polymerization.

### BRIEF DESCRIPTION OF THE DRAWINGS

Figure 1 is a flow chart for one process of the present invention.
Figure 2 is a flow chart for an alternative process of the present invention.
Figure 3 is a graph showing the effect of arsine treatment on acetylene converter performance.

### DETAILED DESCRIPTION

In one aspect, the invention comprises a process for the catalytic and selective removal of acetylene from a gas, for example ethylene, containing acetylene in the presence of a hydrogenation catalyst, such as a noble metal, for example, palladium, by passing the ethylene containing the acetylene together with an inert gas containing an amount of an acetylene converter moderator selected from the group consisting of arsine and phosphine, to prevent temperature runaway while maintaining acceptable activity of the hydrogenation catalyst with which it comes into contact during acetylene hydrogenation.

In one embodiment the hydrogenation catalyst is exposed to arsine in a concentration within the range of 1 to 5 wppb by blending a high concentration of arsine in an inert gas, such as argon.

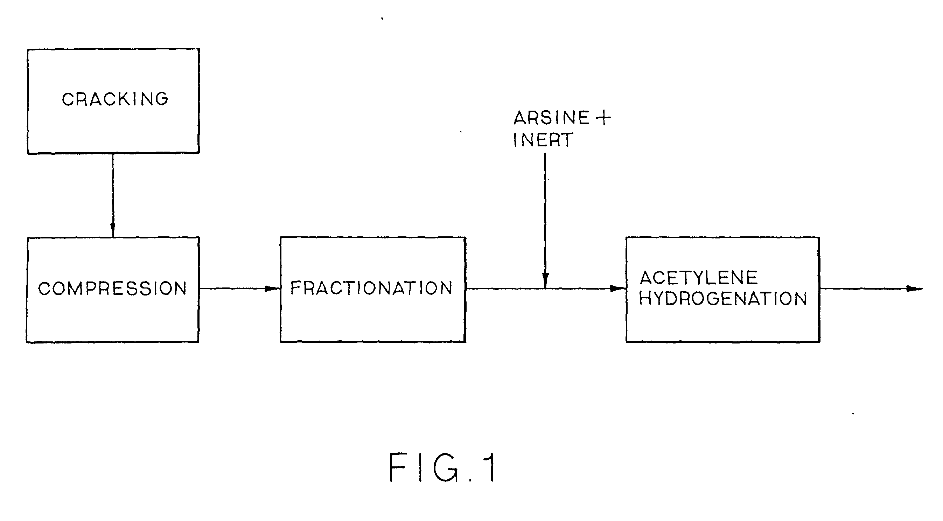

In general, the present invention can be practiced by having 1 to 5 wppb arsine in the feed to the acetylene hydrogenation unit. The preferred way of controllably achieving 1 to 5 wppb arsine in the feed is through the instrumented computer controlled, i.e., injection of a small stream of arsine blended in an inert gas, for example, as shown in Fig. 1. The preferred level of arsine is within the range of 1 wppb to 3 wppb, and this value is controlled to achieve optimum selectivity throughout operation between regenerations.

As the catalyst "ages" through combination of on-stream operation and successive regenerations, the level of required arsine may be varied. For illustration, when operating with new catalyst, the level of arsine in the converter feed is preferably about 5 ppb to achieve nearly optimum selectivity. However, after the catalyst has been subjected to three or more regenerations, the preferred level of arsine may decline to 1 ppb - 3 ppb.

Trace amounts of arsine may be achieved in the feed to the acetylene hydrogenation unit by processing of arsine containing streams within the hydrocarbon, e.g., ethylene, plant process upstream of the acetylene hydrogenation unit. An example of this would be where an arsine containing refinery gas stream, i.e., C₃ and higher, is mixed into the ethylene plant process gas from the steam cracking furnaces.

More specifically, the process of the present invention involves contacting a gas, such as ethylene containing acetylene and the moderator arsine in concentrations within the range of 1 to 5 wppb under conditions to permit hydrogenation of the acetylene, i.e., under controlled acetylene removal conditions, with an appropriate hydrogenation catalyst, for example, a noble metal catalyst such as palladium, until the activity of the catalyst for acetylene removal has been undesirably reduced as a result of deactivation or activity loss by coking and/or green oil formation.

Although the prior art, for example, US-A-4,227,025, indicates that the presence of arsine in the gas otherwise composed of ethylene and acetylene will cause the hydrogenation catalyst to become severely deactivated, it has been unexpectedly discovered that this is not the case where, in accordance with the present invention, arsine is used at concentrations within the range of 1 to 5 wppb.

For purposes of the present invention, therefore, it is important to precisely control the amount of arsine to within the range of 1 to 5 wppb which requires extremely sensitive and accurate analytical techniques. Three analytical techniques are preferred for detecting trace levels of phosphine and arsine required to practice the present invention. The analytical techniques preferred for purposes of the present invention are:
a) The MDA analyser
   model 7100 lab
   model 8500 on-line
   This analyzer is supplied commercially by MDA Scientific, Lincolnshire, Illinois
b) The Silver diethyldithiocarbamate method is a slightly modified form of British Standards Method BS 4404; 1968

For purposes of the present invention, inasmuch as the gas is arsine, then the potassium iodide/stannous chloride followed by zinc and acid may be deleted from the standard procedure otherwise described in BS 4404; 1968, because these steps would convert organic arsenic to arsine. By passing high volumes of gas through the silver diethyldithiocarbamate solution, low detection levels can be obtained.

Thus, in contrast to what is taught or suggested by the prior art, it has been unexpectedly discovered that at such low levels, arsenic does not appear to build up on the palladium sites causing catalyst deactivation. Although deactivation of the catalyst may occur to some extent, activity loss which results in the deactivation of the catalyst is believed to be a result of coking and/or green oil formation.

In this regard, it has been discovered that hydrogenation catalysts suitable for purposes of the present invention can tolerate an amount up to about 2 wt.% arsenic before permanent deactivation occurs.

For purposes of the present invention, such low levels of arsenic can be detected by the following procedure:

Fresh Pd on Al₂O₃ catalyst was impregnated with arsenic to a variety of concentrations by contacting the catalyst with an aqueous solution of arsenic pentoxide and drying. Each impregnated catalyst was regenerated in a muffle furnace to simulate transfer of arsenic to the base. The acetylene hydrogenation ability of each regenerated catalyst was determined by passing a gas containing acetylene, hydrogen, ethylene, and nitrogen, over the catalyst and measuring activity as a function of temperature.

The results are tabulated below:

**Table 1**

| Effect of Arsenic Loading on Catalyst Activity | |
|---|---|
| Activity temperature (°C) for 90% Acetylene Removal | Arsenic loading on Catalyst (wt.%) |
| 57 | 0.0 |
| 58 | 0.25 |
| 60 | 0.80 |
| 64 | 1.15 |
| 67 | 1.25 |
| 68 | 1.40 |

The results indicate that no activity loss was observed until the arsenic concentration exceeded 0.75 wt.%. Above this level, the activity dropped rapidly. When the loading reached 2%, the activity has been determined to be generally unacceptable for a commercial operation.

As a practical matter, it has been discovered that the catalyst activity will reach an unacceptable level as a result of successive regenerations before the catalyst will become permanently poisoned by the arsenic as a result of treatment in accordance with the present invention. In this regard, lab simulations show that following regeneration, permanent loss in activity starts to occur at arsenic loadings above 0.75 wt.%, and becomes very significant at 2 wt.% loading.

For purposes of such simulations in accordance with the present invention, the following procedure was used.

An acetylene containing gas blend was fed at a known flow rate from a cylinder to the reactor. Inside the reactor was an arsenic promoted (moderated) palladium on alumina catalyst. Each batch of catalyst was prepared by contacting the catalyst with an aqueous solution of arsenic pentoxide, evaporating the residual water, drying and regenerating at 450°C in air. The gas rate was chosen such that the space velocity was 5000 v/hr/v. The products from the reactor were analyzed via GC to determine the extent of acetylene hydrogenation.

In a typical experiment, the extent of acetylene removal was determined as a function of reactor temperature and arsenic content on the catalyst bed. The relative catalyst activity was then determined as a function of the arsenic loading.

In accordance with the present invention, the gas, e.g., ethylene containing acetylene and arsine in concentrations within the range of 1 wppb to 5 wppb under controlled acetylene removal conditions, is contacted with an appropriate hydrogenation catalyst preferably by either an external injection of arsine technique or a controlled leakage of indigenous arsine through an arsine removal bed.

In either case, the process parameters are essentially the same, the main difference being the manner the arsine enters the gas stream to the acetylene converter. The converter operating conditions are:

| | |
|---|---|
| feed composition | acetylene 1.0% |
| ethylene | 65.0% |
| ethane | 34.0% |
| arsine | 2 ppb |
| catalyst | 0.03%Pd on Al₂O₃ |
| temperature | 48.9°C (120°F) |
| pressure | 2.07 MPa (300 psig) |
| space velocity | 3000 v/hr./v |

For the direct injection technique, arsine is injected into the process stream from cylinders containing 200 ppm arsine in ethylene at a controlled rate such that the 2 ppb concentration in the process gas is achieved.

For the controlled leakage technique, an upstream arsine removal bed, i.e., PbO on Al₂O₃, is operated in such a manner as to allow 2 ppb arsine to leak into the process stream as measured by an on-line analyzer.

In both cases, the acetylene converter operates at high selectivity, i.e., 0.75% absolute ethylene gain and acceptable catalyst activity.

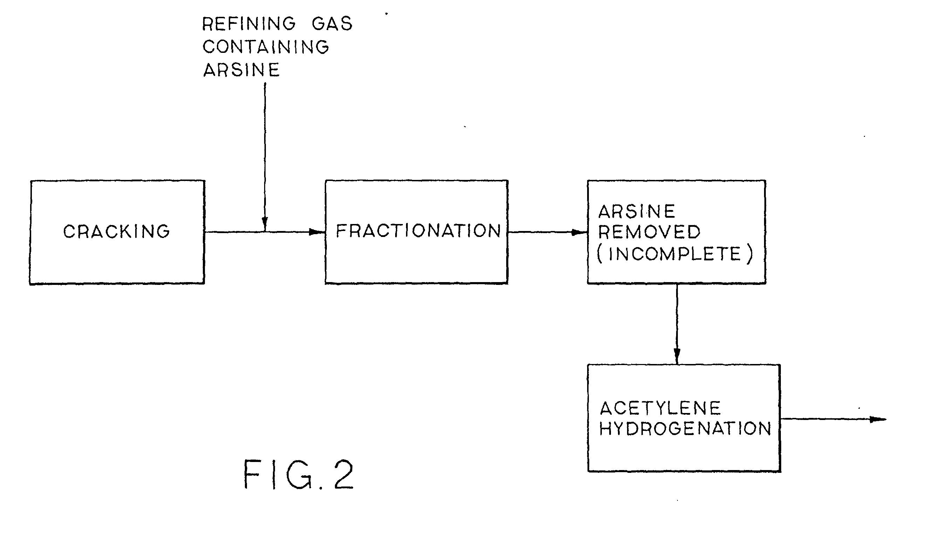

In an alternative embodiment for practicing the present invention, for example, as shown in Fig. 2, the refining gas containing arsine is introduced into the stream upstream of the fractionation stage. Such schemes may be varied with respect to the location of the refinery gas injection, H₂S/CO₂ clean-up of the refinery gas and the like.

Once the activity of the catalyst is observed as being reduced, for example, by coking and/or green oil formation, the catalyst may then be subjected to an appropriate regeneration procedure.

Where appropriate, regeneration of the catalyst may be conducted by heating the reactor in the presence of steam and air to about 399°C to 538°C (750°F - 1000°F) for several hours, and subsequently cooling the reactor under an inert atmosphere, e.g., nitrogen.

### EXAMPLES

### Example I

The following example is representative of the process of the present invention as applied to a gas containing ethylene and acetylene which also includes arsine as an acetylene converter moderator.

Typical operating conditions of the Example are designated (1), with the preferred levels designated (2):

| | |
|---|---|
| Temperature: | (1) 26°C-177°C (80°F-350°F) |
| | (2) 48.9°C-107°C (120°F-225°F) |
| Pressure: | (1) 689 kPag-5.17 MPag (100 psig-750 psig) |
| | (2) 2.07 MPag-3.10 MPag (300 psig-450 psig) |
| Space Velocity | (1) 500 v/hr./v-15,000 v/hr./v |
| | (2) (800 v/hr./v-3,000 v/hr.v) |

### Example II

The following example is representative of a procedure in accordance with the present invention which is believed to show that arsenic is transferred from the palladium sites to the catalyst base during regeneration.

Fresh Pd on Al₂O3 catalyst was impregnated with arsenic to a variety of concentrations by contacting the catalyst with an aqueous solution of arsenic pentoxide and drying. Each impregnated catalyst was regenerated in a muffle furnace to simulate transfer of arsenic to the base. The acetylene hydrogenation ability of each regenerated catalyst was determined by passing a gas containing acetylene, hydrogen, ethylene, and nitrogen, and measuring activity as a function of temperature.
These results are tabulated below:

**Table 2**

| Effect of Arsenic Loading on Catalyst Activity | |
|---|---|
| Temperature °C for 90% Acetylene Removal | Wt.% Arsenic on Catalyst |
| 57 | 0.0 |
| 58 | 0.5 |
| 60 | 0.75 |
| 65 | 1.15 |
| 67 | 1.25 |
| 69 | 1.40 |

The results indicate that no significant activity loss was observed until the arsenic concentration exceeded 0.75 wt.%. Above this level, the activity dropped rapidly. When the loading reached 2%, the activity was considered unacceptable for a commercial operation.

The catalyst activity towards acetylene hydrogenation is evidenced by a drop in operating temperature from end-of-run (EOR), which is typically 121°C (250°F) to start-of-run (SOR) which is typically 48.9°C (120°F).

Also this example shows that greater than 1.25% permanently deactivates the catalyst but that a catalyst having an arsine content within the range of less than about 0.01% to about 0.75% is regenerable.

Accordingly, the arsenic does not tend to buildup on the palladium sites causing catalyst deactivation.

### Example III

As indicated above, it has been discovered that arsenic does not build up on the palladium sites of the catalyst because arsenic is transferred from the palladium sites to the catalyst base during regeneration. Therefore, the catalyst deactivation which may be experienced as a result of activity loss is due to coking and/or green oil formation which has been discovered to precede arsenic poisoning.

The following example is presented to evidence this phenomenon.

Typical and preferred operating conditions of temperature, pressure and space velocity are as shown in Example I.

The feed gas containing 1 ppb - 2 ppb of arsine is passed over the catalyst at the preferred operating conditions. The temperature is progressively raised to compensate for the activity loss caused by green oil/polymer build-up. This results in a loss of selectivity. Eventually, the selectivity becomes unsatisfactory and this run is terminated. The arsenic loading is calculated to be much less than 0.75 wt.%, activity and selectivity can be recovered by regeneration. The process is then repeated.

### EXAMPLE IV

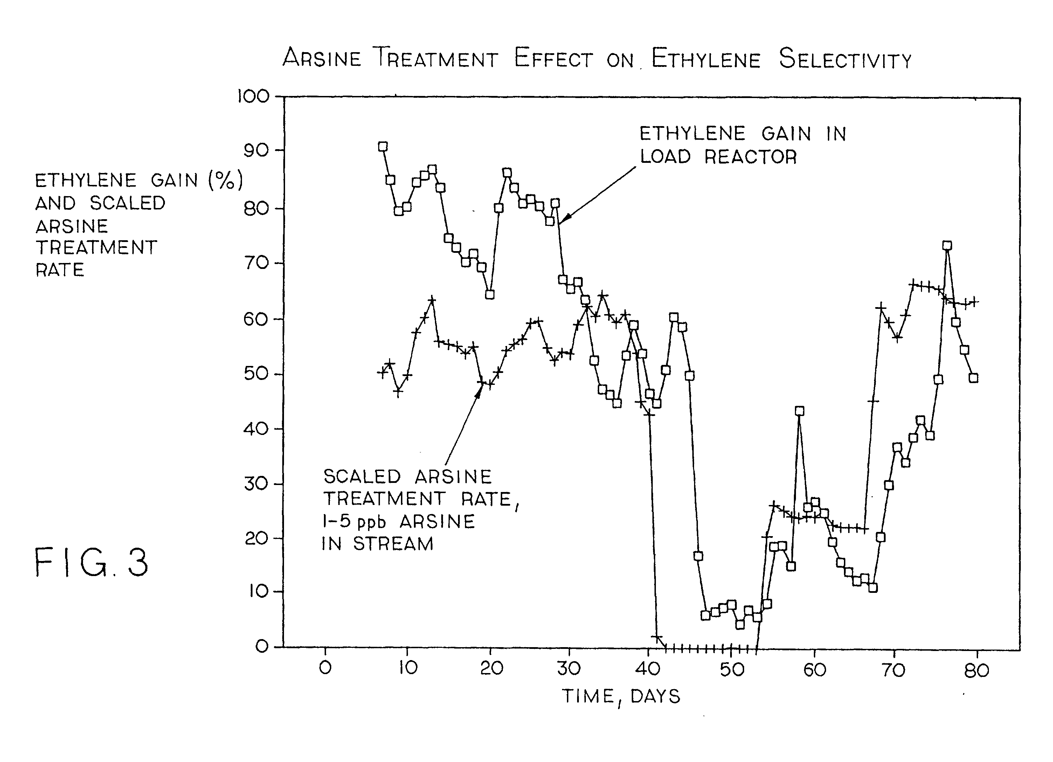

The following example shows the effects of arsine treatment on acetylene converter performance. The data are depicted in the graph shown in Figure 3.

The operating parameters for the data shown in Figure 3 are: gas hourly space velocity (GHSV): 4500 - 6000 v/v/hr.; inlet temperature: 48.9°C - 71.1°C (120 - 160°F); H₂/C₂H₂:1.1 - 2.0.

As shown on the graph, the arsine treatment rate and the reactive selectivity to acetylene are compared. The selectivity is labeled acetylene gain. In acetylene converters, there are two competing reactions: i) acetylene reacts with hydrogen to form ethylene, and ethylene reacts with hydrogen to form ethane. The desired reaction is the reaction that forms ethylene. The ethylene gain is the net per cent of acetylene that forms ethylene.

Related to this, the following equation is used to calculate the ethylene gain:$\text{ethylene gain =} \frac{\text{Moles Ethylene Formed-Moles Ethylene Destroyed}}{\text{Moles Acetylene Reacted}} \text{* 100}$

The graph in Figure 3 shows results for approximately one quarter, i.e., about 80 days, of acetylene converter operation. As can be seen, the ethylene gain trends the arsine treatment rate very well. Before being removed completely, the ethylene gain showed slight variations corresponding to slight variations in the treatment rate. The effect was most noticeable when the treatment was completely removed. When the treatment was reapplied, the converter operation responded proportionately.

## Claims

1. A process for removing acetylene from a hydrocarbon stream, said stream comprising an acetylene converter moderator, by contacting the stream with an acetylene hydrogenation catalyst under conditions effective to permit acetylene hydrogenation, **characterized in that** the moderator is arsine which is introduced into the stream and controlled to give a concentration in the stream of from 1 to 5 wppb prior to contact with the catalyst.

2. The process of claim 1, wherein the concentration of moderator, is from 1 to 3 wppb.

3. The process of any preceding claim wherein the moderator is introduced into the stream in a carrier gas containing up to 1000 wppm moderator.

4. The process of claim 3, wherein carrier gas is introduced in to stream using a direct external injection technique or a controlled leakage technique.

5. The process of claim 3 or 4, wherein the carrier gas containing up to 400 wppm, preferably from 10 wppm - 400 wppm, of moderator.

6. The process of claim 4 or 5, wherein the carrier gas comprises ethylene, ethane, nitrogen, helium, argon, or a mixture of two or more thereof.

7. The process of any preceding claim, wherein the conditions effective to support acetylene hydrogenation comprise a temperature of from 26°C to 177°C (80°F to 350°F) and/or a pressure of from 689 kPa to 5.17 MPa (100 psi to 750 psi) and/or an hourly space velocity of from 3,000 to 15,000 v/hr./v.

8. The process of any of claims 3 to 7, wherein the catalyst is contacted with the carrier gas containing from 100 wppm - 20,000 wppm acetylene at a temperature of from 26°C to 177°C (80°F to 350°F), a pressure of from 689 kPa to 5.17 MPa (100 psi to 750 psi) and at a gas hourly space velocity of from 3000 v/hr./v. to 15,000 v/hr.v. to control the amount of moderator on the catalyst to from 1 wppb to 3wppb.

9. The process of any preceding claim, wherein the deactivation of the hydrogenation catalyst as indicated by a loss of catalyst activity occurs primarily due to coking, green oil formation, or a combination of coking and green oil formation and the loss of catalyst activity due to presence of arsine and/or phosphine is substantially avoided.

10. The process of any preceding claim wherein the catalyst has been regenerated, optionally repeatedly, to restore at least some of the catalyst activity lost due to previous use of the catalyst in the process.

11. The process of claim 10, wherein the moderator comprises arsine and repeatedly regenerated catalyst is used, provided that the activity or selectivity of the catalyst is greater than that of the catalyst subjected to permanent poisoning by arsenic.

12. The process of any preceding claim, wherein the catalyst comprises a transition metal selected from the group consisting of Group VIII metals, Group VIIIA metals and IB metals, optionally based on a support.

13. The process of claim 12, wherein the metal is palladium.

14. The process of claim 13, wherein the support comprises silica, zeolite, alumina, or a combination of two or more thereof.

15. The process of any preceding claim, wherein the hydrocarbon stream comprises ethylene, or a mixture of ethylene and ethane.

16. The process of any preceding claim, wherein the hydrocarbon stream is produced by steam cracking, catalytic cracking, or coking.

17. The process of any preceding claim, which produces a hydrogenated stream substantially free of acetylene and optionally substantially free of carbon monoxide arsine or phosphine.

## Patentansprüche

1. Verfahren zum Entfernen von Acetylen aus einem Acetylenumwandlungsmoderator umfassenden Kohlenwasserstoffstrom, bei dem der Strom mit Acetylen-Hydrierkatalysator unter wirksamen Bedingungen kontaktiert wird, um die Hydrierung von Acetylen zu ermöglichen, **dadurch gekennzeichnet, dass** der Moderator Arsin ist, das in den Strom eingebracht und so eingestellt wird, dass eine Konzentration in dem Strom von 1 bis 5 Gew.ppb vor Kontakt mit dem Katalysator erhalten wird.

2. Verfahren nach Anspruch 1, bei dem die Konzentration des Moderators 1 bis 3 Gew.ppb beträgt.

3. Verfahren nach einem der vorhergehenden Ansprüche, bei dem der Moderator in einem Trägergas in den Strom eingebracht wird, das bis zu 1000 Gew.ppm Moderator enthält.

4. Verfahren nach Anspruch 3, bei dem das Trägergas unter Verwendung einer direkten externen Injektionstechnik oder einer kontrollierten Lecktechnik in den Strom eingebracht wird.

5. Verfahren nach Anspruch 3 oder 4, bei dem das Trägergas bis zu 400 Gew.ppm, vorzugsweise 10 Gew.ppm bis 400 Gew.ppm Moderator enthält.

6. Verfahren nach Anspruch 4 oder 5, bei dem das Trägergas Ethylen, Ethan, Stickstoff, Helium, Argon oder eine Mischung aus zwei oder mehr derselben umfasst.

7. Verfahren nach einem der vorhergehenden Ansprüche, bei dem die zum Unterstützen der Hydrierung von Acetylen wirksamen Bedingungen eine Temperatur von 26°C bis 177°C (80°F bis 350°F) und/oder einen Druck von 689 kPa bis 5,17 MPa (100 psi bis 750 psi) und/oder eine stündliche Raumgeschwindigkeit von 3000 bis 15 000 V/h/V umfassen.

8. Verfahren nach einem der Ansprüche 3 bis 7, bei dem der Katalysator mit dem Trägergas, das 100 Gew.ppm bis 20 000 Gew.ppm Acetylen enthält, bei einer Temperatur von 26°C bis 177°C (80°F bis 350°F), einem Druck von 689 kPa bis 5,17 MPa (100 psi bis 750 psi) und mit einer stündlichen Raumgeschwindigkeit von 3000 V/h/V bis 15 000 V/h/V kontaktiert wird, um die Menge an Moderator, bezogen auf den Katalysator, auf 1 Gew.ppb bis 3 Gew.ppb einzustellen.

9. Verfahren nach einem der vorhergehenden Ansprüche, bei dem die Deaktivierung des Hydrierkatalysators, die sich durch Verlust der Katalysatoraktivität zeigt, hauptsächlich infolge von Verkoken, Grünöibildung oder einer Kombination von Verkoken und Grünöibildung stattfindet und der Verlust der Katalysatoraktivität infolge der Gegenwart von Arsin und/oder Phosphin im Wesentlichen vermieden wird.

10. Verfahren nach einem der vorhergehenden Ansprüche, bei dem der Katalysator gegebenenfalls wiederholt regeneriert worden ist, um mindestens einen Teil der Katalysatoraktivität wieder herzustellen, die durch vorhergehende Verwendung des Katalysators in dem Verfahren verloren gegangen ist.

11. Verfahren nach Anspruch 10, bei dem der Moderator Arsin umfasst und wiederholt regenerierter Katalysator verwendet wird, vorausgesetzt, dass die Aktivität oder Selektivität des Katalysators größer als diejenige des Katalysators ist, der permanenter Vergiftung durch Arsen ausgesetzt worden ist.

12. Verfahren nach einem der vorhergehenden Ansprüche, bei dem der Katalysator Übergangsmetall ausgewählt aus der Gruppe bestehend aus Gruppe VIII Metallen, Gruppe VIIIA Metallen und IB Metallen gegebenenfalls aufgebracht auf Träger umfasst.

13. Verfahren nach Anspruch 12, bei dem das Metall Palladium ist.

14. Verfahren nach Anspruch 13, bei dem der Träger Siliciumdioxid, Zeolith, Aluminiumoxid oder eine Kombination aus zwei oder mehr derselben umfasst.

15. Verfahren nach einem der vorhergehenden Ansprüche, bei dem der Kohlenwasserstoffstrom Ethylen oder eine Mischung aus Ethylen und Ethan umfasst,

16. Verfahren nach einem der vorhergehenden Ansprüche, bei dem der Kohlenwasserstoffstrom durch Dampfcracken, katalytisches Cracken oder Verkoken hergestellt wird.

17. Verfahren nach einem der vorhergehenden Ansprüche, das einen hydrierten Strom erzeugt, der im Wesentlichen frei von Acetylen und gegebenenfalls im Wesentlichen frei von Kohlenmonoxid, Arsin oder Phosphin ist.

## Revendications

1. Procédé pour éliminer l'acétylène d'un courant d'hydrocarbures, ledit courant comprenant un modérateur de convertisseur d'acétylène, en mettant en contact le courant avec un catalyseur d'hydrogénation d'acétylène dans des conditions efficaces pour permettre l'hydrogénation de l'acétylène, **caractérisé en ce que** le modérateur consiste en arsine qui est introduite dans le courant et régulée pour parvenir à une concentration dans le courant de 1 à 5 parties par milliard en poids avant le contact avec la catalyseur.

2. Procédé suivant la revendication 1, dans lequel la concentration de modérateur est comprise dans l'intervalle de 1 à 3 parties par milliard en poids.

3. Procédé suivant l'une quelconque des revendications précédentes, dans lequel le modérateur est introduit dans le courant dans un gaz servant de véhicule contenant jusqu'à 1000 parties par million en poids de modérateur.

4. Procédé suivant la revendication 3, dans lequel le gaz servant de véhicule est introduit dans le courant en utilisant une technique d'injection externe directe ou une technique de fuite contrôlée.

5. Procédé suivant la revendication 3 ou 4, dans lequel le gaz servant de véhicule contient jusqu'à 400 parties par million en poids, de préférence 10 parties par million en poids à 400 parties par million en poids de modérateur.

6. Prccédé suivant la revendication 4 ou 5, dans lequel le gaz servant de véhicule comprend l'éthylène, l'éthane, l'azote, l'hélium, l'argon ou un mélange de deux ou plus de deux d'entre eux.

7. Procédé suivant l'une quelconque des revendications précédentes, dans lequel les conditions efficaces pour assurer l'hydrogénation de l'acétylène comprennent une température comprise dans l'intervalle de 26°C à 177°C (80°F à 350°F) et/ou une pression comprise dans l'intervalle de 689 kPa à 5,17 MPa (100 psi à 750 psi) et/ou une vitesse spatiale horaire comprise dans l'intervalle de 3000 à 15 000 v/h/v.

8. Procédé suivant l'une quelconque des revendications 3 à 7, dans lequel le catalyseur est mis en contact avec le gaz servant de véhicule contenant 100 parties par million en poids à 20 000 parties par million en poids d'acétylène à une température comprise dans l'intervalle de 26°C à 177°C (80°F à 350°F), une pression comprise dans l'intervalle de 689 kPa à 5,17 MPa (100 psi à 750 psi) et une vitesse spatiale horaire de gaz comprise dans l'intervalle de 3000 v/h/v à 15 000 v/h/v pour ajuster la quantité de modérateur sur le catalyseur dans l'intervalle de 1 partie par milliard en poids à 3 parties par milliard en poids.

9. Procédé suivant l'une quelconque des revendications pécédentes, dans lequel la désactivation du catalyseur d'hydrogénation, indiquée par une perte d'activité du catalyseur, se produit principalement en raison d'une cokéfaction, de la formation d'huile verte, d'une association de cokéfaction et de formation d'huile verte, et la perte d'activité du catalyseur due à la présence d'arsine et/ou de phosphine est pratiquement évitée.

10. Procédé suivant l'une quelconque des revendications précédentes, dans lequel le catalyseur a été régénéré, facultativement de manière répétée, pour rétablir au moins une partie de l'activité du catalyseur perdue due à l'utilisation préalable du catalyseur dans le procédé.

11. Procédé suivant la revendication 10, dans lequel le modérateur comprend une arsine et un catalyseur régénéré de manière répétée est utilisé, sous réserve que l'activité ou la sélectivité du catalyseur soit supérieure à celle du catalyseur soumis à un empoisonnement permanent par l'arsenic.

12. Procédé suivant l'une quelconque des revendications précédentes, dans lequel le catalyseur comprend un métal de transition choisi dans le groupe consistant en les métaux du Groupe VIII, les métaux du Groupe VIIIA et les métaux du Groupe IB, facultativement sur un support.

13. Procédé suivant la revendication 12, dans lequel le métal est le palladium.

14. Procédé suivant la revendication 13, dans lequel le support comprend la silice, une zéolite, l'alumine ou une association de deux ou plus de deux d'entre eux.

15. Procédé suivant l'une quelconque des revendications précédentes, dans lequel le courant d'hydrocarbures comprend l'éthylène ou un mélange d'éthylène et d'éthane.

16. Procédé suivant l'une quelconque des revendications précédentes, dans lequel le courant d'hydrocarbures est produit par craquage à la vapeur d'eau, craquage catalytique ou cokéfaction.

17. Procédé suivant l'une quelconque des revendications précédentes, qui produit un courant hydrogéné pratiquement dépourvu d'acétylène et facultativement dépourvu de monoxyde de carbone, d'arsine ou de phosphine.
